(19) Europäisches Patentamt / European Patent Office / Office européen des brevets

(11) **EP 3 906 431 B1**

(12) **FASCICULE DE BREVET EUROPEEN**

(45) Date de publication et mention de la délivrance du brevet:
**23.11.2022 Bulletin 2022/47**

(21) Numéro de dépôt: **19818136.4**

(22) Date de dépôt: **17.12.2019**

(51) Classification Internationale des Brevets (IPC):
*G01V 1/157* (2006.01)    *E21B 28/00* (2006.01)
*G10K 15/06* (2006.01)    *A61B 17/225* (2006.01)
*A61B 17/22* (2006.01)    *E21B 43/00* (2006.01)
*B21D 26/12* (2006.01)

(52) Classification Coopérative des Brevets (CPC):
**E21B 28/00; A61B 17/225; G01V 1/157;
G10K 15/06;** A61B 17/22022; B21D 26/12;
E21B 43/003

(86) Numéro de dépôt international:
**PCT/EP2019/085609**

(87) Numéro de publication internationale:
**WO 2020/141068 (09.07.2020 Gazette 2020/28)**

(54) **DISPOSITIF DE DÉCHARGE ÉLECTRIQUE IMPULSIONNELLE**

GEPULSTE ELEKTRISCHE ENTLADUNGSVORRICHTUNG

PULSED ELECTRIC DISCHARGE DEVICE

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **31.12.2018 FR 1874395**

(43) Date de publication de la demande:
**10.11.2021 Bulletin 2021/45**

(73) Titulaire: **ADM28 France
31100 Toulouse (FR)**

(72) Inventeur: **PECQUOIS, Romain
31270 VILLENEUVE-TOLOSANE (FR)**

(74) Mandataire: **Argyma
14 Boulevard de Strasbourg
31000 Toulouse (FR)**

(56) Documents cités:
**EP-A2- 2 274 741      WO-A1-2010/146016
US-A1- 2005 113 722    US-A1- 2012 157 892
US-A1- 2014 074 111**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

### Domaine technique

La présente invention se rapporte au domaine de l'électronique de puissance et concerne plus particulièrement un dispositif et un procédé de décharge électrique impulsionnelle dans un liquide. De tels procédé et dispositif trouvent notamment leur application au formage électrohydraulique, aux outils sismiques, à la stimulation de puits de pétrole ou à la lithotripsie.

### Technique antérieure

En électronique de puissance, il est connu d'utiliser un dispositif de décharge électrique impulsionnelle permettant de transférer en une fraction de seconde un courant de très haute intensité sous très haute tension entre deux électrodes immergées dans un liquide. La tension entre les deux électrodes est fournie par une alimentation externe, comprise par exemple entre 1 et 40 kV, comprenant des modules capacitifs permettant de stocker l'énergie électrique et de la restituer en une fraction de seconde sous la forme d'un courant de très haute intensité sous très haute tension.

De manière connue, lors d'une décharge électrique impulsionnelle à haute tension dans un liquide, par exemple dans de l'eau, on distingue deux phases : une première phase, dite « de chauffage » ou « pré-décharge », suivie d'une deuxième phase dite de « claquage ». La phase de chauffage est déclenchée par la mise sous tension de l'une des électrodes à une première valeur de tension élevée, par exemple de 20 kV, l'autre électrode étant reliée à une masse fixant la référence de potentiel, par exemple de 0 kV. Lors de la phase de chauffage, la tension définie entre les deux électrodes permet de chauffer le liquide jusqu'à ébullition afin de créer un canal gazeux permettant de créer les conditions de claquage. La fourniture d'énergie au liquide entraine une chute de tension aux bornes des électrodes jusqu'à une deuxième valeur de tension pour laquelle l'énergie fournie au liquide est suffisamment importante pour déclencher le claquage. Le claquage correspond à une décharge électrique se propageant dans le canal gazeux et créant un arc électrique permettant la circulation du courant entre les deux électrodes.

Le document US 2005/0113722 propose un dispositif de décharge électrique impulsionnelle dans un liquide, comprenant deux électrodes immergées dans un liquide, un générateur de tension et un module de contrôle configuré pour mesurer le courant et la tension de claquage entre les électrodes, l'écartement des électrodes et pour comparer les valeurs mesurées avec des courbes ou valeurs de référence, ensuite le module ajuste l'alimentation en tension pour maintenir une décharge optimale au claquage.

Cependant, ce type de dispositif présente plusieurs inconvénients, notamment dans son application au formage électrohydraulique d'une pièce métallique. Tout d'abord, le niveau de tension au moment de la création de l'arc n'est pas maitrisé. L'énergie concentrée entre les électrodes, qui est ensuite convertie en onde de pression, n'est donc pas constante d'un tir à un autre. Dans son application au formage, il en résulte que, d'un essai à l'autre, la pression de formage appliquée sur la pièce n'est pas identique. Or, de telles différences de formage peuvent se révéler trop importantes de sorte qu'une partie des pièces produites ne soit pas conforme.

### Exposé de l'invention

La présente invention a pour but de remédier au moins en partie à ces inconvénients en proposant une solution simple, fiable et efficace de dispositif de décharge électrique impulsionnelle.

À cet effet, l'invention a tout d'abord pour objet un dispositif de décharge électrique impulsionnelle dans un liquide, ledit dispositif comprenant au moins une paire d'électrodes, configurées pour être immergées dans ledit liquide, et un générateur de tension, apte à appliquer une tension de chauffage entre les électrodes de ladite au moins une paire d'électrodes pendant une durée dite « de chauffage » afin de chauffer le liquide jusqu'à provoquer une décharge électrique impulsionnelle dans ledit liquide, ledit dispositif étant remarquable en ce qu'il comprend en outre un module de contrôle configuré pour :

- commander le générateur de tension à partir d'une consigne de tension de chauffage prédéterminée afin que ledit générateur de tension applique une tension de chauffage entre les électrodes de ladite au moins une paire d'électrodes pendant la durée de chauffage jusqu'à obtenir une décharge électrique impulsionnelle entre lesdites électrodes,
- mesurer la tension aux bornes des électrodes afin de déterminer la tension, dite « de claquage », à laquelle la décharge électrique impulsionnelle s'est produite,
- estimer la quantité d'énergie fournie au liquide pendant la durée de chauffage, dite « quantité d'énergie de chauffage », à partir de la consigne de tension de chauffage prédéterminée et de la tension de claquage mesurée,
- déterminer une nouvelle consigne de tension de chauffage à appliquer entre les électrodes de ladite au moins une paire d'électrodes à la prochaine décharge électrique impulsionnelle à partir de la quantité d'énergie de chauffage estimée et d'une consigne de tension de claquage prédéfinie.

Le dispositif selon l'invention permet d'appliquer une consigne de tension de chauffage qui est fonction à la fois de la quantité d'énergie dissipée lors de la décharge électrique impulsionnelle précédente et des pertes énergétiques générées pendant la durée de chauffage du tir précédent, lesdites pertes étant proportionnelles à la tension de claquage. Une telle consigne permet de contrôler avec précision le niveau de tension au moment du tir. L'énergie concentrée entre les électrodes, qui est ensuite convertie en onde de pression, est ainsi sensiblement constante d'un tir à un autre. Dans son application au formage, il en résulte que, d'un essai à l'autre, la pression de formage appliquée sur la pièce est sensiblement identique, rendant ainsi les pièces conformes.

Selon un aspect de l'invention, le dispositif est configuré pour déterminer la nouvelle consigne de tension de chauffage en calculant la médiane ou la moyenne des quantités d'énergie de chauffage préalablement estimées par le dispositif lors du ou des tirs précédents.

La nouvelle consigne de tension de chauffage peut être déterminée à partir de la médiane ou de la moyenne des quantités d'énergie de chauffage préalablement estimées aux N dernières itérations, N étant un nombre entier naturel, de préférence impair et par exemple supérieur ou égal à 3.

La consigne de tension de claquage peut être ajustée d'un tir à un autre, par exemple manuellement par un opérateur ou bien automatiquement en fonction d'une valeur cible à atteindre au fil des tirs.

Selon une caractéristique de l'invention, le dispositif comprend un module de mesure telle que, par exemple une sonde ou un capteur, configuré pour mesurer la tension de claquage aux bornes des électrodes.

Avantageusement, le module de contrôle est configuré pour calculer la quantité d'énergie de chauffage à partir de la tension de claquage mesurée au dernier tir et de la consigne de tension de chauffage appliquée au dernier tir selon la formule suivante :

$$E_{loss} = \frac{1}{2} \times C \times (V_C^2 - V_B^2)$$

où $E_{loss}$ est la quantité d'énergie de chauffage, C correspond à la capacité du générateur de tension, $V_C$ est la consigne de tension de chauffage appliquée lors du dernier tir et $V_B$ est la tension de claquage mesurée lors du dernier tir.

Selon un aspect de l'invention, le module de contrôle est configuré pour déterminer la nouvelle consigne de tension de chauffage à appliquer entre les électrodes au prochain tir à partir de la valeur de quantité d'énergie de chauffage estimée lors du dernier tir, ou de la médiane calculée le cas échéant, et de la consigne de tension de claquage prédéfinie, par exemple manuellement par un opérateur ou automatiquement de manière à atteindre une valeur cible, selon la formule suivante :

$$V_C = \sqrt{\left( \frac{2E_{loss}}{C} + V_{B\_CONS}^2 \right)}$$

où Vc est la nouvelle consigne de tension de chauffage calculée, $E_{loss}$ est la valeur de quantité d'énergie de chauffage déterminée lors du dernier tir et $V_{B\_CONS}$ est la consigne de tension de claquage visée.

De préférence, la durée de chauffage est comprise entre 5 μs et 500 ms.

De préférence encore, la tension de claquage est comprise entre 1 et 40 kV.

Selon une caractéristique de l'invention, suite au démarrage du dispositif, la valeur initiale de la consigne de tension de chauffage est déterminée à partir d'une quantité d'énergie de chauffage prédéterminée et d'une valeur de tension de claquage prédéterminée, par exemple stockées dans une zone mémoire du dispositif.

Dans une forme de réalisation, le générateur de tension comprend un module capacitif, relié à l'une des électrodes de l'au moins une paire d'électrodes, comprenant par exemple un ou plusieurs condensateurs.

L'invention concerne également un procédé de génération d'une décharge électrique dans un liquide à partir d'un dispositif de décharge électrique impulsionnelle tel que présenté précédemment, ledit procédé, mis en œuvre par le module de contrôle, étant remarquable en ce qu'il comprend les étapes de :

- commande du générateur de tension à partir d'une consigne de tension de chauffage prédéterminée afin que ledit générateur de tension applique une tension de chauffage entre les électrodes de ladite au moins une paire d'électrodes jusqu'à obtenir une décharge électrique impulsionnelle entre lesdites électrodes,
- mesure de la tension aux bornes des électrodes afin de déterminer la tension, dite « de claquage », à laquelle la décharge électrique impulsionnelle s'est produite,
- estimation de la quantité d'énergie fournie au liquide pendant la durée de chauffage, dite « quantité d'énergie de chauffage », à partir de la consigne de tension de chauffage prédéterminée et de la tension de claquage mesurée,
- détermination d'une nouvelle consigne de tension de chauffage à appliquer entre les électrodes de ladite au moins une paire d'électrodes à la prochaine décharge électrique impulsionnelle à partir de la quantité d'énergie de chauffage estimée et d'une consigne de tension de claquage prédéfinie.

Selon un aspect de l'invention, le procédé est répété une pluralité de fois et la nouvelle consigne de tension de chauffage est déterminée à chaque itération à partir de la quantité d'énergie de chauffage estimée à l'itération précédente ou des quantités d'énergie de chauffage estimées aux itérations précédentes.

De manière préférée, la nouvelle consigne de tension de chauffage est déterminée à partir de la médiane ou de la moyenne des quantités d'énergie de chauffage préalablement estimées aux itérations précédentes.

La nouvelle consigne de tension de chauffage peut être déterminée à partir de la médiane ou de la moyenne des quantités d'énergie de chauffage préalablement estimées aux N dernières itérations, N étant un nombre entier naturel, par exemple supérieur ou égal à 3.

De préférence, suite au démarrage du dispositif, le procédé est tout d'abord répété au moins dix fois afin de calibrer le dispositif pour permettre des tirs ultérieurs à des consignes précises, permettant notamment d'atteindre une valeur de tension de claquage cible.

Avantageusement, la quantité d'énergie de chauffage est déterminée à partir de la tension de claquage mesurée au dernier tir et de la consigne de tension de chauffage prédéterminée appliquée au dernier tir selon la formule suivante :

$$E_{loss} = \frac{1}{2} \times C \times (V_C^2 - V_B^2)$$

où $E_{loss}$ est la quantité d'énergie de chauffage, C correspond à la capacité du générateur de tension, $V_C$ est la consigne de tension de chauffage appliquée lors du dernier tir et $V_B$ est la tension de claquage mesurée lors du dernier tir.

Selon un aspect de l'invention, la nouvelle consigne de tension de chauffage à appliquer entre les électrodes au prochain tir est calculée à partir de la valeur de quantité d'énergie de chauffage estimée, ou de la médiane calculée le cas échéant, et de la consigne de tension de claquage prédéfinie, par exemple manuellement par un opérateur ou automatiquement de manière à atteindre une valeur cible, selon la formule suivante :

$$V_C = \sqrt{\left(\frac{2E_{loss}}{C} + V_{B\_CONS}^2\right)}$$

où Vc est la nouvelle consigne de tension de chauffage calculée, $E_{loss}$ est la valeur de quantité d'énergie de chauffage déterminée lors du dernier tir et $V_{B\_CONS}$ est la consigne de tension de claquage visée.

D'autres caractéristiques et avantages de l'invention apparaîtront lors de la description qui suit faite en regard des figures annexées et données à titre d'exemples non limitatifs et dans lesquelles des références identiques sont données à des objets semblables.

La figure 1 illustre schématiquement une forme de réalisation du dispositif de décharge électrique impulsionnelle selon l'invention.

La figure 2 illustre schématiquement un mode de réalisation du procédé selon l'invention.

Le dispositif selon l'invention permet de réaliser des décharges électriques impulsionnelles dans un liquide, par exemple de l'eau ou du sang. Le dispositif selon l'invention peut notamment être utilisé pour réaliser du formage électrohydraulique de pièces métalliques, pour réaliser de la prospection géophysique en générant des ondes sismiques, pour améliorer la production de puits de pétrole (stimulation) ou encore pour traiter des calculs rénaux par lithotripsie.

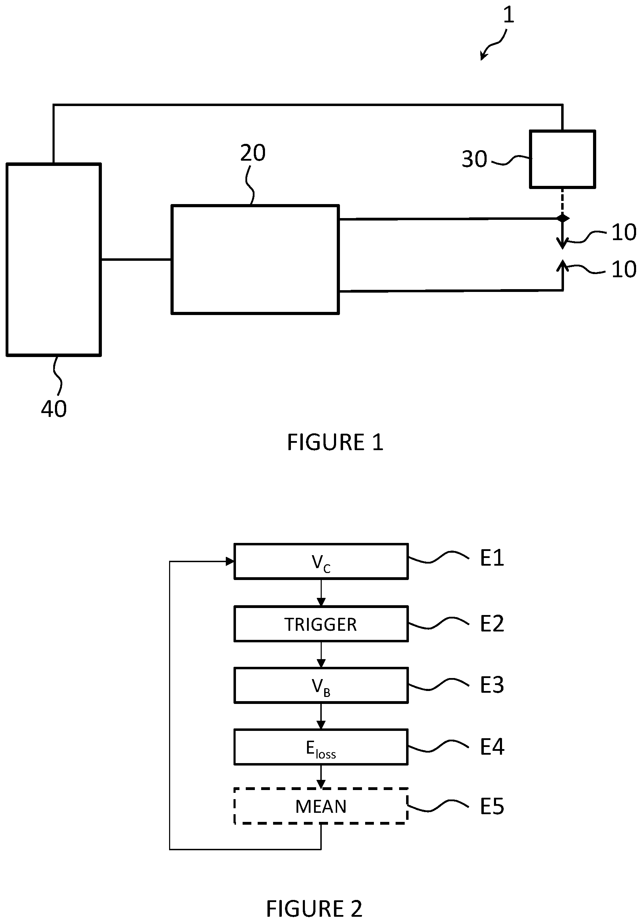

On a représenté à la figure 1 une forme de réalisation du dispositif 1 selon l'invention. Dans cette forme de réalisation, le dispositif 1 comprend une unique paire d'électrodes 10, un générateur de tension 20, un module de mesure 30 et un module de contrôle 40. On notera que dans une autre forme de réalisation, le dispositif 1 pourrait comprendre plus d'une paire d'électrodes 10.

La paire d'électrodes 10 est configurée pour être immergée dans le liquide dans lequel on veut réaliser une série de décharges électriques impulsionnelles. Plus précisément, la paire d'électrodes 10 est configurée pour recevoir une tension appliquée entre les deux électrodes 10 par le générateur de tension 20 pendant une durée dite « de chauffage » afin de chauffer le liquide jusqu'à générer une décharge électrique impulsionnelle provoquant un arc électrique entre les deux électrodes 10. La tension pour laquelle la décharge électrique impulsionnelle se produit est appelée « tension de claquage ». L'électrode 10 qui est connectée à la borne positive du générateur de tension 20 est dite « de charge » tandis que l'électrode 10 qui est connectée à la borne négative du générateur de tension 20 est dite « de réception ».

Lors d'un tir, les charges électriques se propagent depuis l'électrode 10 de charge vers l'électrode 10 de réception dans le volume de liquide et de gaz les séparant. Les électrodes 10 peuvent par exemple être de forme générale cylindrique creuse, symétrique de révolution. De préférence, les électrodes 10 sont alignées "bout à bout", c'est-à-dire agencées de sorte que leurs axes longitudinaux respectifs (non représentés) coïncident et qu'elles présentent des extrémités libres éloignées d'une distance fixe prédéterminée, par exemple comprise entre 1 et 50 mm environ, préférentiellement entre 1 et 25 mm (l'augmentation de la distance entre les électrodes 10 permettant d'augmenter l'énergie déposée entre lesdites électrodes 10), et en regard l'une de l'autre selon la direction axiale (direction des axes de symétrie). Lors d'une décharge électrique impulsionnelle, l'arc électrique se produit entre ces extrémités libres, dont les faces extrêmes annulaires en regard sont sensiblement planes (dans des plans transversaux).

Le générateur de tension 20 est configuré pour appliquer une tension de chauffage entre les électrodes 10 à partir d'une consigne de tension de chauffage $V_C$ prédéterminée fournie par le module de contrôle 40 jusqu'au déclenchement d'une décharge électrique impulsionnelle dans le liquide. La durée pendant laquelle la tension de chauffage $V_C$ est appliquée entre les électrodes 10 est appelée « durée de chauffage ».

Le générateur de tension 20 peut se présenter sous la forme d'une pluralité de condensateurs ou d'un générateur de tension à courant constant. L'utilisation de plusieurs condensateurs permet de fournir une tension de l'ordre de quelques kilovolts en un temps très court, par exemple de l'ordre de 10 ms.

Le module de mesure 30 permet de mesurer la tension de claquage à laquelle la décharge électrique impulsionnelle se produit lors d'un tir. Le module de mesure 30 peut par exemple se présenter sous la forme d'une sonde ou d'un capteur de mesure de tension.

Le module de contrôle 40 est configuré pour commander le générateur de tension 20 à partir d'une valeur de consigne de tension de chauffage prédéterminée afin que ledit générateur de tension 20 applique la tension correspondant à cette consigne entre les électrodes 10 pendant la durée de chauffage afin de provoquer une décharge électrique impulsionnelle entre lesdites électrodes 10. Plus précisément, le module de contrôle 40 est configuré pour envoyer une consigne de tension de chauffage au générateur de tension 20 de sorte qu'à réception de ladite consigne, le générateur de tension 20 applique la valeur de tension correspondant à cette consigne entre les électrodes 10 afin de déclencher une décharge électrique impulsionnelle entre lesdites électrodes. De préférence, la durée de chauffage est comprise entre 5 µs et 500 ms et la tension de décharge est comprise entre 1 et 40 kV.

Le module de contrôle 40 est configuré pour collecter les mesures de tension réalisées par le module de mesure 30 et pour estimer la quantité d'énergie fournie au liquide pendant la durée de chauffage, appelée « quantité d'énergie de chauffage » $E_{loss}$, à partir de la consigne de tension de chauffage $V_C$ prédéterminée appliquée au dernier tir et de la valeur de tension de claquage $V_B$ mesurée au dernier tir.

Le module de contrôle 40 est configuré pour déterminer une nouvelle consigne de tension de chauffage $V_C$ à appliquer entre les électrodes 10 afin de réaliser la prochaine décharge électrique impulsionnelle.

Le module de contrôle 40 est configuré pour mesurer la tension aux bornes des électrodes afin de déterminer la tension de claquage $V_B$, pour laquelle la décharge électrique impulsionnelle se produit lors d'un tir.

Le module de contrôle 40 est configuré pour calculer la quantité d'énergie de chauffage $E_{loss}$ à partir de la tension de claquage $V_B$ mesurée et de la consigne de tension de chauffage $V_C$ prédéterminée, appliquée lors du dernier tir qu'il a commandé via le générateur de tension 20, selon la formule suivante :

$$E_{loss} = \frac{1}{2} \times C \times (V_C^2 - V_B^2)$$

où C est la valeur de capacité des condensateurs du générateur de tension 20.

Au démarrage du dispositif 1, la consigne de tension de chauffage est déterminée à partir d'une quantité d'énergie de chauffage $E_{loss}$ initiale prédéterminée et d'une consigne de tension de claquage $V_{B\_CONS}$ prédéfinie permettant une décharge électrique impulsionnelle entre les électrodes 10. Une fois qu'au moins un tir a été effectué, la nouvelle consigne de tension de chauffage est déterminée à partir de la ou des dernières quantités d'énergie de chauffage $E_{loss}$ calculées par ledit module de contrôle 40.

De manière préférée, le dispositif est configuré pour déterminer la nouvelle consigne de tension de chauffage $V_C$ en calculant la médiane ou la moyenne de quantités d'énergie de chauffage $E_{loss}$ préalablement estimées. La nouvelle consigne de tension de chauffage peut être déterminée à partir de la médiane ou de la moyenne des quantités d'énergie de chauffage $E_{loss}$ préalablement estimées aux N dernières itérations, N étant un nombre entier naturel, de préférence impair et par exemple supérieur ou égal à 3. De préférence, le module de contrôle 40 est configuré pour recalculer la médiane ou la moyenne des quantités d'énergie de chauffage $E_{loss}$ après chaque décharge électrique impulsionnelle.

Le module de contrôle 40 peut par exemple comprendre un calculateur, un processeur ou un microcontrôleur afin de réaliser les différentes tâches susmentionnées.

Un exemple de mise en œuvre du dispositif 1 va maintenant être décrit en référence à la figure 2.

Le module de contrôle 40 calcule tout d'abord dans une étape E1 une valeur de consigne de tension de chauffage $V_C$ à appliquer pour déclencher le prochain tir à partir d'une valeur de quantité d'énergie de chauffage $E_{loss}$ et d'une valeur de tension de claquage $V_B$ selon la formule suivante :

$$E_{loss} = \frac{1}{2} \times C \times (V_C^2 - V_B^2)$$

Au démarrage du dispositif 1, la valeur de quantité d'énergie de chauffage prédéterminée $E_{loss}$ initiale et la valeur de la consigne de tension de claquage $V_{B\_CONS}$ initiale, sont stockées dans une zone mémoire du module de contrôle 40 ou saisies manuellement par un opérateur.

La consigne de tension de chauffage $V_C$ est calculée par le module de contrôle 40 selon la formule suivante :

$$V_C = \sqrt{\left(\frac{2E_{loss}}{C} + V_{B\_CONS}^2\right)}$$

Le module de contrôle 40 commande ensuite le générateur de tension 20 afin que ledit générateur de tension 20 applique, dans une étape E2, la consigne de tension de chauffage $V_C$, déterminée à l'étape E1, entre les électrodes 10 jusqu'à obtenir une décharge électrique impulsionnelle entre lesdites électrodes 10 (TRIGGER).

Lors de ce tir, le module de mesure 30 mesure, dans une étape E3, la tension de claquage $V_B$ aux bornes des électrodes 10 et envoie cette mesure au module de contrôle 40.

Le module de contrôle 40 calcule alors dans une étape E4 une estimation de la quantité d'énergie de chauffage $E_{loss}$ utilisée lors du dernier tir à partir de la tension de claquage $V_B$ mesurée à l'étape E3 et de la consigne de tension de chauffage $V_C$ utilisée à l'étape E2 selon la formule suivante :

$$E_{loss} = \frac{1}{2} \times C \times (V_C^2 - V_B^2)$$

où $V_C$ est la consigne de tension de chauffage et $V_B$ est la valeur de tension de claquage mesurée à l'étape E3.

De préférence, le module de contrôle 40 calcule alors dans une étape E5 (optionnelle) la médiane MEAN des quantités d'énergie de chauffage $E_{loss}$ estimées aux tirs précédents, si elles sont disponibles.

Le module de contrôle 40 détermine ensuite une nouvelle consigne de tension de chauffage $V_C$ à appliquer entre les électrodes 10 au prochain tir en réitérant l'étape E1 à partir de la valeur de quantité d'énergie de chauffage $E_{loss}$ estimée, ou de la médiane MEAN calculée le cas échéant, et de la consigne de tension de claquage $V_{B\_cons}$ prédéfinie, par exemple manuellement par un opérateur ou automatiquement de manière à atteindre une valeur de tension de claquage cible entre les électrodes d'un tir à l'autre, selon la formule suivante :

$$V_C = \sqrt{\left( \frac{2E_{loss}}{C} + V_{B\_CONS}^2 \right)}$$

où $E_{loss}$ est la valeur de quantité d'énergie de chauffage déterminée à l'étape E4 et $V_B$ est la consigne de tension de claquage visée.

Le module de contrôle 40 recommence les étapes E1 à E4 (ou E5) afin de réaliser une série de décharges électriques impulsionnelles à énergie sensiblement constante.

Suite au démarrage du dispositif 1, le procédé peut être mis en œuvre plusieurs fois afin de calibrage le dispositif 1 et d'obtenir des consignes de tension de chauffage $V_C$ précises pour les tirs suivants, qui peuvent alors servir en production, par exemple pour du formage électrohydraulique.

L'invention permet donc avantageusement de définir des consignes de tension de chauffage précises et affinées afin de permettre une régularité des tirs en termes de tension de claquage. Il est à noter que la présente invention n'est pas limitée aux exemples décrits ci-dessus et est susceptible de nombreuses variantes accessibles à l'homme de l'art, l'invention étant définie dans les revendications.

## Revendications

1. Dispositif (1) de décharge électrique impulsionnelle dans un liquide, ledit dispositif (1) comprenant au moins une paire d'électrodes (10), configurées pour être immergées dans ledit liquide, et un générateur de tension (20), apte à appliquer une tension de chauffage ($V_C$) entre les électrodes (10) de ladite au moins une paire d'électrodes (10) pendant une durée dite « de chauffage » afin de chauffer le liquide jusqu'à provoquer une décharge électrique impulsionnelle dans ledit liquide, ledit dispositif (1) étant **caractérisé en ce qu'**il comprend en outre un module de contrôle (40) configuré pour :

    - commander le générateur de tension (20) à partir d'une consigne de tension de chauffage prédéterminée afin que ledit générateur de tension (20) applique une tension de chauffage ($V_C$) entre les électrodes (10) de ladite au moins une paire d'électrodes (10) pendant la durée de chauffage jusqu'à obtenir une décharge électrique impulsionnelle entre lesdites électrodes (10),
    - mesurer la tension aux bornes des électrodes (10) afin de déterminer la tension, dite « de claquage » ($V_B$), à laquelle la décharge électrique impulsionnelle s'est produite,

le dispositif étant **caractérisé en ce que** le module de contrôle est en outre configuré pour:

    - estimer la quantité d'énergie fournie au liquide pendant la durée de chauffage, dite « quantité d'énergie de chauffage » ($E_{loss}$), à partir de la consigne de tension de chauffage ($V_C$) prédéterminée et de la tension de claquage ($V_B$) mesurée,
    - déterminer une nouvelle consigne de tension de chauffage ($V_C$) à appliquer entre les électrodes (10) de ladite au moins une paire d'électrodes (10) à la prochaine décharge électrique impulsionnelle à partir de la quantité d'énergie de chauffage ($E_{loss}$) estimée et d'une consigne de tension de claquage ($V_{B\_cons}$) prédéfinie.

2. Dispositif (1) selon la revendication 1, ledit dispositif (1) étant configuré pour déterminer la nouvelle consigne de tension de chauffage ($V_C$) en calculant la médiane ou la moyenne des quantités d'énergie de chauffage ($E_{loss}$) préalablement estimées par le dispositif (1) lors du ou des tirs précédents.

3. Dispositif (1) selon la revendication précédente, dans lequel la nouvelle consigne de tension de chauffage ($V_C$) est déterminée à partir de la médiane des quantités d'énergie de chauffage ($E_{loss}$) préalablement estimées aux N dernières itérations, N étant un nombre entier naturel impair supérieur ou égal à 3.

4. Dispositif (1) selon l'une des revendications précédentes, comprenant un module de mesure (30) configuré pour mesurer la tension de claquage aux bornes des électrodes (10).

5. Dispositif (1) selon l'une des revendications précédentes, dans lequel le module de contrôle (40) est configuré pour calculer la quantité d'énergie de chauffage ($E_{loss}$) à partir de la tension de claquage ($V_B$) mesurée au dernier tir et de la consigne de tension de chauffage ($V_C$) appliquée au dernier tir selon la formule : $E_{loss} = \frac{1}{2} \times C \times (V_C^2 - V_B^2)$ où $E_{loss}$ est la quantité d'énergie de chauffage, C correspond à la capacité du générateur de tension, $V_C$ est la consigne de tension de chauffage appliquée lors du dernier tir et $V_B$ est la tension de claquage mesurée.

6. Dispositif (1) selon l'une des revendications précédentes, dans lequel le module de contrôle (40) est configuré pour déterminer la nouvelle consigne de tension de chauffage ($V_C$) à appliquer entre les électrodes (10) au prochain tir à partir de la valeur de quantité d'énergie de chauffage ($E_{loss}$) estimée lors du dernier tir, ou de la médiane calculée le cas échéant, et de la consigne de tension de claquage ($V_{B\_CONS}$) prédéfinie, selon la formule suivante:

$$V_C = \sqrt{\left(\frac{2E_{loss}}{C} + V_{B\_CONS}^2\right)}.$$

7. Procédé de génération d'une décharge électrique dans un liquide à partir d'un dispositif (1) de décharge électrique impulsionnelle selon l'une des revendications précédentes, ledit procédé, mis en œuvre par le module de contrôle (40), étant **caractérisé en ce qu'**il comprend les étapes de :

- commande (E1) du générateur de tension (20) à partir d'une consigne de tension de chauffage ($V_C$) prédéterminée afin que ledit générateur de tension (20) applique une consigne de tension de chauffage ($V_C$) prédéterminée entre les électrodes (10) de ladite au moins une paire d'électrodes (10) jusqu'à obtenir une décharge électrique impulsionnelle entre lesdites électrodes (10),
- mesure (E2) de la tension aux bornes des électrodes (10) afin de déterminer la tension, dite « de claquage » ($V_B$), à laquelle la décharge électrique impulsionnelle s'est produite, et

**caractérisé en ce qu'**il comprend en outre les étapes de:

- estimation (E3) de la quantité d'énergie fournie au liquide pendant la durée de chauffage, dite « quantité d'énergie de chauffage » ($E_{loss}$), à partir de la consigne de tension de chauffage ($V_C$) prédéterminée et de la tension de claquage ($V_B$) mesurée,
- détermination (E4) d'une nouvelle consigne de tension de chauffage ($V_C$) à appliquer entre les électrodes (10) de ladite au moins une paire d'électrodes (10) à la prochaine décharge électrique impulsionnelle à partir de la quantité d'énergie de chauffage ($E_{loss}$) estimée et d'une consigne de tension de claquage ($V_{B\_cons}$) prédéfinie.

8. Procédé selon la revendication 7, dans lequel, ledit procédé étant répété une pluralité de fois, la nouvelle consigne de tension de chauffage ($V_C$) est déterminée à chaque itération à partir de la ou des quantités d'énergie de chauffage ($E_{loss}$) estimées à l'itération précédente ou aux itérations précédentes.

9. Procédé selon l'une quelconque des revendications 7 et 8, dans lequel la nouvelle consigne de tension de chauffage ($V_C$) est déterminée à partir de la médiane des quantités d'énergie de chauffage ($E_{loss}$) préalablement estimées aux itérations précédentes.

10. Procédé selon l'une quelconque des revendications 7 à 9, dans lequel la quantité d'énergie de chauffage ($E_{loss}$) est déterminée à partir de la consigne de tension de chauffage ($V_C$) prédéterminée appliquée au dernier tir et de la tension de claquage ($V_B$) mesurée au dernier tir selon la formule suivante :

$E_{loss} = \frac{1}{2} \times C \times (V_C^2 - V_B^2)$ où C correspond à la capacité du générateur de tension (20).

**Patentansprüche**

1. Vorrichtung (1) zur gepulsten elektrischen Entladung in einer Flüssigkeit, wobei die Vorrichtung (1) mindestens ein Paar Elektroden (10) umfasst, die ausgelegt sind, um in die Flüssigkeit eingetaucht zu sein, und einen Spannungsgenerator (20), der imstande ist, eine Heizspannung (Vc) zwischen den Elektroden (10) des mindestens einen Paars Elektroden (10) während einer als "Heizdauer" bezeichneten Dauer anzulegen, um die Flüssigkeit zu heizen, bis eine gepulste elektrische Entladung in der Flüssigkeit erfolgt, wobei die Vorrichtung (1) **dadurch gekennzeichnet ist, dass** sie ferner ein Steuermodul (40) umfasst, das ausgelegt ist, um:

- den Spannungsgenerator (20) ausgehend von einem vorher festgelegten Sollwert der Heizspannung zu steuern, damit der Spannungsgenerator (20) eine Heizspannung (Vc) zwischen den Elektroden (10) des mindestens einen Paars Elektroden (10) während der Heizdauer bis zum Erhalt einer gepulsten elektrischen Entladung zwischen den Elektroden (10) anlegt,
- die Spannung an den Klemmen der Elektroden (10) zu messen, um die als "Durchschlagspannung" ($V_B$) bezeichnete Spannung zu bestimmen, bei der die gepulste elektrische Entladung stattfindet,

wobei die Vorrichtung **dadurch gekennzeichnet ist, dass** das Steuermodul ferner ausgelegt ist, um:

- die der Flüssigkeit während der Heizdauer bereitgestellte Energiemenge, bezeichnet als "Heizenergiemenge" ($E_{loss}$) ausgehend vom vorher festgelegten Sollwert der Heizspannung (Vc) und von der gemessenen Durchschlagspannung ($V_B$) zu schätzen,
- einen neuen Sollwert der Heizspannung (Vc) zu bestimmen, die zwischen den Elektroden (10) des mindestens einen Paars Elektroden (10) bei der nächsten gepulsten elektrischen Entladung ausgehend von der geschätzten Heizenergiemenge ($E_{loss}$) und einem vorher festgelegten Durchschlagspannungs-Sollwert ($V_{B\_cons}$) anlegen ist.

2. Vorrichtung (1) nach Anspruch 1, wobei die Vorrichtung (1) ausgelegt ist, um den neuen Sollwert der Heizspannung (Vc) durch Berechnung des Medians oder des Mittels der Heizenergiemengen ($E_{loss}$) zu bestimmen, die zuvor von der Vorrichtung (1) bei dem oder den vorherigen Schüssen geschätzt wurden.

3. Vorrichtung (1) nach vorangehendem Anspruch, wobei der neue Sollwert der Heizspannung (Vc) ausgehend vom Median der Heizenergiemengen ($E_{loss}$) bestimmt wird, die zuvor bei den N letzten Wiederholungen geschätzt wurden, wobei N eine ungerade natürliche Ganzzahl größer oder gleich 3 ist.

4. Vorrichtung (1) nach einem der vorangehenden Ansprüche, die ein Messmodul (30) umfasst, das ausgelegt ist, um die Durchschlagspannung an den Klemmen der Elektroden (10) zu messen.

5. Vorrichtung (1) nach einem der vorangehenden Ansprüche, wobei das Steuermodul (40) ausgelegt ist,

um die Heizenergiemenge ($E_{loss}$) ausgehend von der Durchschlagspannung ($V_B$), die beim letzten Schuss gemessen wurde, und vom Sollwert der Heizspannung (Vc), der auf den letzten Schuss angewendet wurde, gemäß der Formel

$$E_{loss} = \frac{1}{2} \times C \times (V_C^2 - V_B^2)$$

zu berechnen, wobei $E_{loss}$ die Heizenergiemenge ist, C der Kapazität des Spannungsgenerators entspricht, Vc der Sollwert der Heizspannung ist, der beim letzten Schuss angewendet wurde und $V_B$ die gemessene Durchschlagspannung ist.

6. Vorrichtung (1) nach einem der vorangehenden Ansprüche, wobei das Steuermodul (40) ausgelegt ist, um den neuen Sollwert der Heizspannung (Vc), die zwischen den Elektroden (10) beim nächsten Schuss anzulegen ist, ausgehend vom Wert der Heizenergiemenge ($E_{loss}$), der beim letzten Schuss geschätzt wurde, oder gegebenenfalls des berechneten Medians und des vorher festgelegen Durchschlagspannungs-Sollwerts ($V_{B\_CONS}$) gemäß der folgenden Formel zu berechnen:

$$V_C = \sqrt{\left(\frac{2E_{loss}}{C} + V_{B\_CONS}^2\right)}.$$

7. Verfahren zur Erzeugung einer elektrischen Entladung in einer Flüssigkeit ausgehend von einer Vorrichtung (1) zur gepulsten elektrischen Entladung nach einem der vorangehenden Ansprüche, wobei das Verfahren, das vom Steuermodul (40) durchgeführt wird, **dadurch gekennzeichnet ist, dass** es die folgenden Schritte umfasst:

- Steuern (E1) des Spannungsgenerators (20) ausgehend von einem vorher festgelegten Sollwert der Heizspannung (Vc), damit der Spannungsgenerator (20) einen vorher festgelegten Sollwert der Heizspannung ($V_C$) zwischen den Elektroden (10) des mindestens einen Paars Elektroden (10) bis zum Erhalt einer gepulsten elektrischen Entladung zwischen den Elektroden (10) anlegt,
- Messen (E2) der Spannung an den Klemmen der Elektroden (10), um die als "Durchschlagspannung" ($V_B$) bezeichnete Spannung zu bestimmen, bei der die gepulste elektrische Entladung stattfindet, und

**dadurch gekennzeichnet, dass** es ferner die folgenden Schritte umfasst:

- Schätzen (E3) der der Flüssigkeit während der Heizdauer bereitgestellten Energiemenge, bezeichnet als "Heizenergiemenge" ($E_{loss}$) ausge-

hend vom vorher festgelegten Sollwert der Heizspannung (Vc) und von der gemessenen Durchschlagspannung (V_B),

- Bestimmen (E4) eines neuen Sollwerts der Heizspannung (Vc), der zwischen den Elektroden (10) des mindestens einen Paars Elektroden (10) bei der nächsten gepulsten elektrischen Entladung ausgehend von der geschätzten Heizenergiemenge (E_loss) und einem vorher festgelegten Durchschlagspannungs-Sollwert (V_B_cons) anzuwenden ist.

8. Verfahren nach Anspruch 7, wobei, wobei das Verfahren eine Vielzahl von Malen wiederholt wird, der neue Sollwert der Heizspannung (Vc) bei jeder Wiederholung ausgehend von der oder den bei der vorherigen Wiederholung oder den vorherigen Wiederholungen geschätzten Heizenergiemenge/n (E_loss) bestimmt wird.

9. Verfahren nach einem der Ansprüche 7 und 8, wobei der neue Sollwert der Heizspannung (Vc) ausgehend vom Median der zuvor bei den vorherigen Wiederholungen geschätzten Heizenergiemengen (E_loss) bestimmt wird.

10. Verfahren nach einem der Ansprüche 7 bis 9, wobei die Heizenergiemenge (E_loss) ausgehend vom zuvor bestimmten Sollwert der Heizspannung (Vc), der auf den letzten Schuss angewendet wurde, und von der Durchschlagspannung (V_B), die beim letzten Schuss gemessen wurde, gemäß der folgenden Formel

$$E_{loss} = \frac{1}{2} \times C \times (V_C^2 - V_B^2)$$ bestimmt wird, wobei C der Kapazität des Spannungsgenerators (20) entspricht.

**Claims**

1. A device (1) for pulsed electric discharge in a liquid, said device (1) comprising at least a pair of electrodes (10), configured to be immersed in said liquid, and a voltage generator (20) capable of applying a heating voltage (Vc) between the electrodes (10) of said at least one pair of electrodes (10) for a so-called "heating" period in order to heat the liquid until a pulsed electric discharge is caused in said liquid, said device (1) being **characterized in that** it further comprises a control module (40) configured to:

   - control the voltage generator (20) from a predetermined heating voltage set point so that said voltage generator (20) applies a heating voltage (Vc) between the electrodes (10) of said at least one pair of electrodes (10) during the heating period until a pulsed electric discharge is obtained between said electrodes (10),
   - measure the voltage across the electrodes (10) in order to determine the breakdown voltage (V_B) at which the pulsed electric discharge occurred, the device being **characterized in that** the control module (40) is further configured to:
   - estimate the quantity of energy supplied to the liquid during the heating period, so-called "quantity of heating energy" (E_loss), from the predetermined heating voltage set point (Vc) and the breakdown voltage (V_B) measured,
   - determine a new heating voltage set point (Vc) to be applied between the electrodes (10) of said at least one pair of electrodes (10) at the next pulsed electric discharge from the heating energy quantity (E_loss) estimated and a predefined breakdown voltage set point (V_B).

2. The device (1) according to claim 1, said device (1) being configured to determine the new heating voltage set point (Vc) by calculating the median or mean of the quantities of heating energy (E_loss) previously estimated by the device (1) during the previous shot(s).

3. The device (1) according to the preceding claim, wherein the new heating voltage set point (Vc) is determined from the median of the quantities of heating energy (E_loss) previously estimated at the last N iterations, N being an odd natural number greater than or equal to 3.

4. The device (1) according to one of the preceding claims, comprising a measurement module (30) configured to measure the breakdown voltage across the electrodes (10).

5. The device (1) according to one of the preceding claims, wherein the control module (40) is configured to calculate the quantity of heating energy (E_loss) from the breakdown voltage (V_B) measured at the last shot and the heating voltage set point (Vc) applied at the last shot according to the formula:

$$E_{loss} = \frac{1}{2} \times C \times (V_C^2 - V_B^2)$$ where E_loss is the quantity of heating energy, C corresponds to the capacitance of the voltage generator, Vc is the heating voltage set point applied at the last shot and V_B is the breakdown voltage measured.

6. The device (1) according to one of the preceding claims, wherein the control module (40) is configured to determine the new heating voltage set point (Vc) to be applied between the electrodes (10) in the next shot from the value of the quantity of heating energy (E_loss) estimated in the last shot, or from the median calculated if necessary, and from the predefined breakdown voltage set point ($V_{B\_CONS}$), according to the following formula:

$$V_C = \sqrt{\left(\frac{2E_{loss}}{C} + V_{B\_CONS}^2\right)}.$$

7. A method for generating an electric discharge in a liquid from a pulsed electric discharge device (1) according to one of the preceding claims, said method, implemented by the control module (40), being **characterized in that** it comprises the steps of:

- controlling (E1) the voltage generator (20) from a predetermined heating voltage set point (Vc) so that said voltage generator (20) applies a predetermined heating voltage set point (Vc) between the electrodes (10) of said at least one pair of electrodes (10) until a pulsed electric discharge is obtained between said electrodes (10),
- measuring (E2) the voltage across the electrodes (10) in order to determine the so-called "breakdown" voltage ($V_B$) at which the pulsed electric discharge occurred, and **characterized in that** it further comprises the steps of:
- estimating (E3) the quantity of energy supplied to the liquid during the heating period, the so-called "quantity of heating energy" ($E_{loss}$), from the predetermined heating voltage set point (Vc) and the breakdown voltage ($V_B$) measured,
- determining (E4) a new heating voltage set point (Vc) to be applied between the electrodes (10) of said at least one pair of electrodes (10) at the next pulsed electric discharge from the quantity of heating energy ($E_{loss}$) estimated and a predefined breakdown voltage set point ($V_B$ set).

8. The method for claim 7, wherein, with said method being repeated a plurality of times, the new heating voltage set point (Vc) is determined at each iteration from the quantity(ies) of heating energy ($E_{loss}$) estimated at the previous iteration or iterations.

9. The method according to any of claims 7 and 8, wherein the new heating voltage set point (Vc) is determined from the median of the quantities of heating energy ($E_{loss}$) previously estimated at the previous iterations.

10. The method according to any of claims 7 to 9, wherein the quantity of heating energy ($E_{loss}$) is determined from the predetermined heating voltage set point (Vc) applied at the last shot and the breakdown voltage ($V_B$) measured at the last shot according to the following formula:

$$E_{loss} = \frac{1}{2} \times C \times (V_C^2 - V_B^2)$$

where C corresponds to the capacitance of the voltage generator (20).

1

20

30

10

10

40

**FIGURE 1**

| $V_C$ | E1 |
|---|---|
| TRIGGER | E2 |
| $V_B$ | E3 |
| $E_{loss}$ | E4 |
| MEAN | E5 |

**FIGURE 2**

**EP 3 906 431 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20050113722 A